# EUROPÄISCHE PATENTSCHRIFT

(11) **EP 1 268 367 B1**
(45) Veröffentlichungstag und Bekanntmachung des Hinweises auf die Patenterteilung: **21.07.2004**
(21) Anmeldenummer: 01936129.4
(22) Anmeldetag: 22.03.2001
(51) Int. Cl.: C07B 41/00, C07C 29/50

(54) **VERFAHREN ZUR OXIDATION VON KOHLENWASSERSTOFFEN**
METHOD FOR OXIDIZING HYDROCARBONS
PROCEDE POUR OXYDER DES HYDROCARBURES

(30) Priorität: 30.03.2000 DE 10015880
(43) Veröffentlichungstag der Anmeldung: 02.01.2003
(73) Patentinhaber: Degussa AG, 40474 Düsseldorf (DE)
(72) Erfinder: KÜHNLE, Adolf, 45770 Marl (DE); DUDA, Mark, 67071 Ludwigshafen (DE); SHELDON, Roger, Arthur, NL-2281 VA Rijswijk (NL); SASIDHARAN, Manickam, Tamil Nadu 606604 (IN); ARENDS, Isabella, W., C., E., NL-2548 SL's Gravenhage (NL); SCHIFFER, Thomas, 45721 Haltern (DE); FRIES, Guido, 45657 Recklinghausen (DE); KIRCHHOFF, Jochen, 59348 Lüdinghausen (DE); JOST, Carsten, Dr., 45770 Marl (DE)
(86) Internationale Anmeldenummer: PCT/EP2001/003289
(87) Internationale Veröffentlichungsnummer: WO 2001/074742

(56) Entgegenhaltungen:
- EP-A- 0 885 868
- US-A- 5 030 739
- MARCH J: "Free-radical substitution" , ADVANCED ORGANIC CHEMISTRY. REACTIONS, MECHANISMS, AND STRUCTURE, NEW YORK, WILEY & SONS, US, PAGE 706 XP002178384 ISBN: 0-471-58148-8 Absatz [0002]
- YASUTAKA ISHII ET AL: "A NOVEL CATALYSIS OF N-HYDROXYPHTHALIMIDE IN THE OXIDATION OF ORGANIC SUBSTRATES BY MOLECULAR OXYGEN" JOURNAL OF ORGANIC CHEMISTRY, AMERICAN CHEMICAL SOCIETY. EASTON, US, Bd. 60, Nr. 13, 30. Juni 1995 (1995-06-30), Seiten 3934-3935, XP000512054 ISSN: 0022-3263 in der Anmeldung erwähnt
- ISHII: "A novel catalysis of NHPI combined with Co(Acac)n (n=2 or 3) in the oxidation of organic substrates with molecular oxygen" J. MOL. CATAL. A, Bd. 117, 1997, Seiten 123-137, XP002178383 in der Anmeldung erwähnt

## Beschreibung

Die Erfindung betrifft ein Verfahren zur katalytischen Oxidation von substituierten oder unsubstituierten Kohlenwasserstoffen zu den entsprechenden Alkoholen, Hydroperoxiden, Carbonsäuren, Dicarbonsäuren oder Ketonen.

Die Oxidation von Kohlenwasserstoffen ist eine wichtige Reaktion in der industriellen organischen Chemie. Für diese Oxidationen können Verbindungen wie KMnO₄, CrO₃ oder HNO₃ als Oxidationsmittel eingesetzt werden. Diese weisen aber einerseits den Nachteil eines relativ hohen Preises, andererseits bringt ihre Verwendung unerwünschte Koppelprodukte mit sich, die Entsorgungsprobleme und eine ökologische Belastung darstellen können.

Bevorzugt werden deshalb Oxidationsmittel auf der Basis von Peroxiden oder N₂O verwendet. Das preiswerteste Oxidationsmittel stellt aber molekularer Sauerstoff, entweder in reiner Form oder als Luftsauerstoff, dar. Sauerstoff für sich allein genommen ist aber für die Oxidation von Kohlenwasserstoffen zumeist nicht geeignet, da die Reaktivität des O₂-Moleküls, das in der energetisch günstigen Triplett-Form vorliegt, nicht ausreichend ist.

Durch den Einsatz von Redoxmetallkatalysatoren ist es möglich, molekularen Sauerstoff für die Oxidation von organischen Verbindungen zu nutzen. Eine ganze Reihe industrieller Verfahren basieren auf der metallkatalisierten Autoxidation von Kohlenwasserstoffen. So erfolgt z. B. die Oxidation von Cyclohexan mit O₂ zu Cyclohexanol bzw. Cyclohexanon unter Verwendung von Kobaltsalzen. Diese industriellen Verfahren basieren auf einem Radikalkettenmechanismus. Das Biradikal Sauerstoff reagiert mit einem Kohlenwasserstoffradikal unter Bildung eines Peroxyradikals und anschliessender Kettenfortpflanzung durch Abstraktion eines H-Atoms an einem weiteren Kohlenwasserstoff. Neben Metallsalzen können aber auch organische Moleküle als Radikalstarter fungieren.

Nachteilig bei diesen Verfahren ist, dass die Selektivität mit steigendem Umsatz sehr stark sinkt und deshalb die Verfahren auf niedrigem Umsatzniveau gefahren werden müssen. So wird beispielweise die Oxidation von Cyclohexan zu Cyclohexanol / Cyclohexanon bei einem Umsatz von 10 bis 12 % durchgeführt, damit die Selektivität 80 bis 85 % beträgt ("Industrielle Organische Chemie" 1994, 261, VCH-Verlag, D-69451 Weinheim). In einem weiteren wichtigen industriellen Autoxidationsprozess, der Cumol-Oxidation, beträgt der Umsatz ca. 30 % bei einer Cumolhydroperoxid-Selektivität von ca. 90% (Loc. cit. 385 ff).

Eine Alternative zu Metallsalzkatalysatoren stellt die Verwendung von Katalysatorsystemen bzw. Mediatorsystemen wie z. B. N-Hydroxyphthalimid (NHPI) dar. Jedoch ist die Reaktionsgeschwindigkeit bei den vorgestellten Verfahren trotz der hohen Menge an Mediator (bis zu äquimolaren Verhältnissen gegenüber dem Substrat) nicht befriedigend (J. Mol. Catalysis A. 1997, *117*, 123 - 137). So beschreibt US 5 030 739 die Verwendung von N-Hydroxydicarbonsäureimiden zur Oxidation von Isoprenderivaten zu den entsprechenden Acroleinverbindungen. Eine kombinierte Oxidation/Dehydration von Cyclohexadienen bzw. Sechsringsystemen wie α-Terpinen führt zwar zum Cumolderivat, welches jedoch nicht weiter oxidiert wird. Dieses Verfahren ist daher zur Umsetzung von Cumol zu Cumolhydroperoxid nicht geeignet.

Im allgemeinen werden Mengen an Mediator von mindestens 10 Mol-% im Verhältnis zum Substrat eingesetzt, wobei höhere Mengen an Mediator zur Steigerung der Reaktionsgeschwindigkeit verwendet werden (*J*. *Org. Chem.* 1995, *60,* 3934-3935). Die Produktselektivität ist für eine technische Anwendung nicht ausreichend. So wird bei der Oxidation von Cumol mit NHPI ein Produktgemisch mit Acetophenon als Hauptprodukt erhalten, das gewünschte Oxidationsprodukt Cumolhydroperoxid konnte jedoch nicht isoliert werden (*J*. *Org*. *Chem*. **1995,** *60*, 3934-3935).

Eine Weiterentwicklung des Systems stellt die Verwendung von Co-Katalysatoren dar. Als Co-Katalysatoren können Metallverbindungen, insbesondere Schwermetallsalze, Enzyme oder starke Brönsted-Säuren verwendet werden. So zeigten Ishii et al., daß NHPI in Verbindung mit Redoxmetallsalzen als Co-Katalysator Vorteile gegenüber der analogen Oxidation ohne Co-Katalysator aufweisen können (z. B. EP 0878234, EP 0864555, EP 0878458, EP 0858835, JP 11180913, J. Mol. Catalysis A. 1997, *117*, 123 - 137). Nachteilig an diesen Systemen ist aber, neben dem unerwünschten Schwermetallgehalt, auch hier die hohe Menge an verwendetem NHPI. Um eine zufriedenstellende Reaktionsgeschwindigkeit zu gewährleisten, müssen mindestens 10 Mol-% an Mediator verwendet werden. Weiterhin nachteilig ist, dass die eingesetzten Redoxmetalle teilweise weitergehende Reaktionen der Produkte katalysieren und so die Selektivität der Reaktion vermindern.

Oxidationsprodukte von gesättigten zyklischen Kohlenwasserstoffen wie zum Beispiel Cyclopentadecanon, Cyclododecanon oder Cyclooctanon stellen wichtige Riechstoffe bzw. Riechstoffvorprodukte dar. An diese Substanzen werden hohe Reinheitsanforderungen gestellt, so dass selbst katalytisch enthaltene Mengen an Metallsalzen große Probleme bereiten. Notwendige Aufarbeitungsschritte wie zum Beispiel Abtrennung der Metallverbindungen und insbesondere die Rückgewinnung der zugesetzten Metallsalze belasten die Effizienz eines metallsalzkatalysierten Verfahrens und die Umwelt.

Es sind auch Verfahren bekannt geworden, die nur einen Mediator ohne Co-Katalysator verwenden. Diese sind jedoch auf die Oxidation von besonders aktivierten Substraten wie Ether, Ester oder Isoprenderivate beschränkt.

So wird bei der Oxidation von Cumol mit dem System NHPI/Kobaltacetat ein Produktgemisch aus Acetophenon (Selektivität 54 %), 2-Phenyl-2-propanol (10 %) und Phenol (17 %) erhalten. Das gewünschte Produkt Cumolhydroperoxid wird nur intermediär gebildet und ist unter den gegebenen Verfahrensbedingungen nicht stabil.

Eine weitere Verfahrensvariante stellt die Verwendung von NHPI in Verbindung mit Alkoholen oder Aldehyden als Co-Katalysator dar (Chem. Commun. 1999, 727 - 728, Tetrahedron Letters 1999, *40*, 2165 - 2168, Chem. Commun. 1997, 447 - 448). Nachteilig an diesen Verfahren ist die Bildung von Koppelprodukten und das verwendete hohe Mediator-Substrat-Verhältnis (10 Mol-%).

In DE 19723890 wird ein Oxidationssystem, bestehend aus einem organischen Mediator und dem Redoxenzym Laccase, für die Herstellung aromatischer und heteroaromatischer Aldehyde und Ketone beschrieben. Auch hier ist die eingesetzte Menge an Mediator sehr hoch. Zudem weist dieses Verfahren durch die Verwendung eines Enzyms ein kompliziertes Reaktionssystem mit einem biologisch notwendigen Puffersystem auf, das die breite Anwendbarkeit dieses Systems einschränkt.

Die Aufgabe der vorliegenden Erfindung bestand darin, ein schwermetallfreies bzw. metallfreies Verfahren für die Oxidation von Kohlenwasserstoffen zu entwickeln, das hohe Selektivitäten bei hohen Umsätzen aufweist.

Überraschenderweise wurde gefunden, daß Verbindungen des Typs 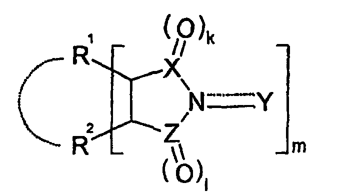 bzw. Derivate dieser Verbindungen auch ohne Schwermetalle oder starke Säuren zur Oxidation von Substraten wie Kohlenwasserstoffen eingesetzt werden können.

Gegenstand der vorliegenden Erfindung ist daher ein Verfahren katalytischen Oxidation von gesättigten oder ungesättigten, substituierten oder unsubstituierten Kohlenwasserstoffen, Heterocyclen, Alkoholen, Ethern, Ketonen, Aldehyden, Aminen oder Russ (im Folgenden: das Substrat) zum entsprechenden Hydroperoxid, Alkohol, Keton, Carbonsäure oder Dicarbonsäure, wobei als Katalysator eine Verbindung der Formel I 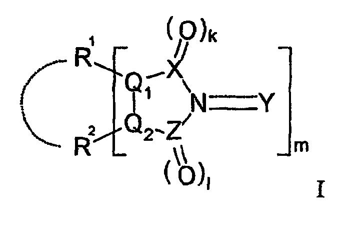 mit
R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können
Q₁, Q₂ = C, CH, N, CR⁵, jeweils gleich oder verschieden
X, Z = C, S, CH₂, jeweils gleich oder verschieden
Y = O, OH
k = 0, 1, 2
l =0, 1,2
m = 1 bis 3;
R⁵ : eine der Bedeutungen von R¹
unter Anwesenheit eines Radikalstarters eingesetzt wird, wobei das molare Verhältnis des Katalysators zum Kohlenwasserstoff zwischen 10⁻⁶ und 2,5 Mol-% liegt.

Im erfindungsgemäßen Verfahren werden keine Metallverbindungen oder Enzyme als Co-Katalysator verwendet. Das Verfahren wird bevorzugt in organischen Lösungsmitteln unter Abwesenheit von starken Säuren durchgeführt, die Verwendung einer wäßrigen Lösung in neutralen bis basischen pH-Bereich ist ebenfalls möglich.

Das molare Verhältnis des Katalysators zu dem zu oxidierenden Kohlenwasserstoff kann in einer speziellen Ausführungsform zwischen 10⁻⁶ und 1 Mol-% liegen.

Die Verwendung des Katalysators (Mediators) gemäß Formel I in diesem geringen Verhältnis zu dem zu oxidierenden Substrat hat überraschenderweise nicht nur das Erreichen hoher Umsätze bei kurzen Reaktionszeiten zur Folge, sondern zeichnet sich auch durch die erreichten Selektivitäten gegenüber dem Stand der Technik aus. Ein weiterer Vorteil des erfindungsgemässen Verfahrens besteht in der Verbesserung der Wirtschaftlichkeit durch die Reduktion der Mediatormenge.

In speziellen Ausführungsformen des erfindungsgemäßen Verfahrens können auch Derivate bzw. Spezialfälte von Verbindungen der Formel I eingesetzt werden.

Bevorzugt sind Mediatoren/Katalysatoren der Formel II, d. h. Verbindungen gemäß Formel I mit m = 1. 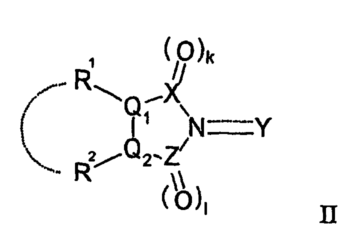 Q¹, Q², R1, R², X, Y, Z, k und l haben die für Formel I definierten Bedeutungen.

Besonders bevorzugt sind Mediatoren/Katalysatoren der Formel ITI 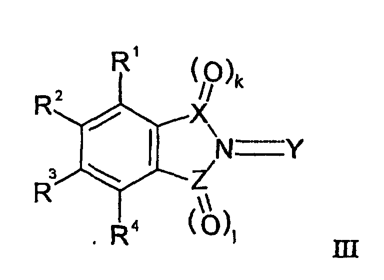 mit
R¹, R², R³, R⁴ = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹, R², R³ und R⁴ identische oder unterschiedliche Reste bezeichnen können, mit
X, Z = C, S, CH₂ jeweils gleich oder verschieden
Y = O, OH
k = 0, 1, 2
l = 0, 1, 2.

Weiterhin kann im erfindungsgemäßen Verfahren ein Hydantoinderivat der Formel IX 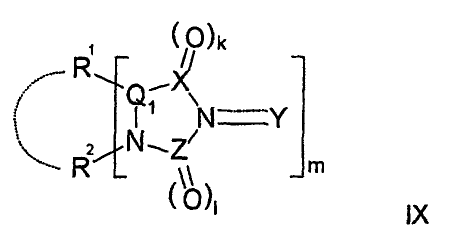 eingesetzt werden,
wobei Q¹, R¹, R², X, Z, k, l, m die im Anspruch 1 genannten Bedeutungen haben.

Beispiele für die im erfindungsgemäßen Verfahren verwendeten Katalysatoren gemäß Formel I sind N-Hydroxyphthalimid, 4-Amino-N-Hydroxyphthalimid, 3-Amino-N-Hydroxyphthalimid, Tetrabromo-N-Hydroxyphthalimid, Tetrachloro-N-Hydroxyphthalimid, N-Hydroxyhetimid, N-Hydroxyhimimid, N-Hydroxytrimellitimid, N-Hydroxy-benzol-1,2,4-tricarbonsäureimid, N,N'-Dihydroxy-pyromellitsäurediimid, N,N'-Dihydroxy-benzophenon-3,3',4,4'tetracarbonsäurediimid, N-Hydroxymaleimid, Pyridin-2,3-dicarbonsäureimid, N-Hydroxysuccinimid, N-Hydroxyweinsäureimid,, N-Hydroxy-5-norbonen-2,3-dicarbonsäureimid, exo-N-Hydroxy-7-oxabicyclo[2.2.1]-hept-5-en-2,3-dicarboximid, N-Hydroxy-cis-cyclohexan-1,2-dicarboximid, N-Hydroxy-cis-4-cyclohexen-1,2-dicarbonsäureimid, N-Hydroxynaphthalsäureimid-Natrium-Salz, Hydantoin, Hydantoinderivate oder N-Hydroxy-o-benzoldisulfonimide.

In einer besonderen Ausführungsform der Erfindung erfolgt die Oxidation in der Flüssigphase bei einer Temperatur von 0 bis 500 °C, bevorzugt 50 bis 300 °C, besonders bevorzugt bei einer Temperatur von 50 bis 200 °C. Dabei kann sowohl ein Lösemittel bzw. Lösemittelgemisch als auch das Substrat selber als Lösemittel verwendet werden.

Die zu oxidierenden Substrate gehören in der Regel zu der Gruppe der substituierten oder unsubstituierten, gesättigten oder ungesättigten Kohlenwasserstoffe. Mit Hilfe des erfindungsgemässen Verfahrens können eine Vielzahl dieser Verbindungen, wie Aromaten, Aliphaten, Olefine oder Alicyclen selektiv oxidiert werden. Die Substrate für das Verfahren der Erfindung können Sauerstoff-, Schwefel- und/oder Stickstoffatome und/oder Cyanogruppen enthalten. Insbesondere kann das Verfahren gemäss der vorliegenden Erfindung zur Oxidation von cyclischen und nicht-cyclischen Alkanen, Alkenen, Alkoholen, Aldehyden, Ketonen, Aminen, Stickstoff enthaltenden heterocyclischen Verbindungen, organischen Sulfiden und Sulfoxiden, Cyanogruppen enthaltende Verbindungen, Benzol, Toluol-Derivaten und Alkylaromaten eingesetzt werden. Beispiele für solche Verbindungen sind insbesondere Ethan, Ethen, Ethin, Propin, Aminocyclododecan, Butadien, Buten, Butylbenzol, Cumol, Cyclodecadien, Cyclodecan, Cyclodecen, Cyclododecadien, Cyclododecan, Cyclododecanol, Cyclohexan, Cyclohexanol, Cyclohexen, Cyclohexanon, Cyclododecanon, Cyclododecatrien, Cyclododecen, Cyclododecylbenzol, Cyclohepten, Cyclohexylbenzol, Cyclononen, Cyclooctadien, Cyclooctan, Cyclooctanol, Cyclooctanon, Cycloocten, Cyclopentadecan, Cyclopentadecanol, Cyclopentadecanon, Cyclopentadecatrien, Cyclopentadecen, Cycolopentadecadien, Decalin, Dicyclododecylether, Ethylbenzol, Isobutan, Isobuten, Isophoron, Isophoronderivate, meta-Xylol, ortho-Xylol, para-Xylol, Picolin, Propan, Propen, Tetralin, Toluol, Trimethylcyclohexan, Trimethylcyclohexanol, Trimethylcyclohexanon, Trivinylcyclohexan, jeweils als Reinstoff, in Lösung oder als Mischung eingesetzt wird.

Bei der Oxidation von cyclischen Verbindungen kann es zur Spaltung des Rings unter Bildung von Dicarbonsäuren, Ketonen, Aldehyden und Alkoholen kommen.

Die Reinigung von Abwässern von diesen Verbindungen durch Oxidation ist mit dem erfindungsgemäßen Verfahren ebenso möglich.

Mit Hilfe dieses Verfahrens kann auch die Oxidation von Wachsen natürlicher und synthetischer Herkunft, insbesondere Kohlenwasserstoffwachse wie Paraffine, Mikrowachse, Polyethylen- und Polypropylenwachse sowie Fischer-Tropsch-Wachse effizient durchgeführt werden. Auch die sogenannte Ammoxidation, wo ein Substrat in Gegenwart von Ammoniak oxididiert wird und dadurch Nitrile hergestellt werden, ist mit Hilfe des vorliegenden Verfahrens möglich.

Im Sinne der vorliegenden Erfindung sollen Russe, die ein Kohlenstoffgerüst mit einem gewissen Anteil an inkorporierten Kohlenwasserstoffen enthalten, als Kohlenwasserstoffe angesehen werden. Die oxidative Oberflächenbehandlung von Russen, die z. B. als Füllstoffe und/oder zur Anfärbung von Polymeren oder Kautschuk verwendet werden, ist ebenfalls eine Einsatzmöglichkeit für das erfindungsgemäße Verfahren. Bei der Oberflächenoxidation von Russen ist sowohl die rein oxidative Oberflächenbehandlung als auch die Ammoxidation von großem technischen Interesse.

Grundsätzlich können mit dem erfindungsgemässen Verfahren durch gezielte Oxidation von Kohlenstoff- oder Heteroatomen Alkohole, Aldehyde, Ketone, Carbonsäuren, Dicarbonsäuren, insbesondere α-ω-Dicarbonsäuren, Epoxide, N-Oxide, Sulfoxide, Sulfone und Sulfonsäuren hergestellt werden.

Bevorzugt wird ein Kohlenwasserstoff mit einem primären, sekundären oder tertiären Kohlenstoffatom zum entsprechenden Hydroperoxid oxidiert. Die Oxidation erfolgt demnach an einer CH-Bindung. Quartäre Kohlenstoffatome, die nur C-C-Bindungen aufweisen, werden durch das erfindungsgemäße Verfahren nicht oxidiert, sondern gespalten. Bevorzugt werden Kohlenwasserstoffe mit einem sekundären oder tertiären Kohlenstoffatom, besonders bevorzugt werden Verbindungen mit einem tertiären Kohlenstoffatom der Formel 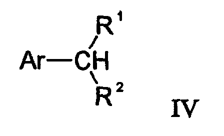 mit
R¹, R² = H, aliphatischer oder aromatischer Kohlenwasserstoffrest mit 1 bis 20 Kohlenstoffatomen, wobei R¹ und R² identische oder unterschiedliche Reste bezeichnen und R¹ und R² über eine kovalente Bindung miteinander verknüpft sein können und
Ar = aromatischer Kohlenwasserstoffrest
als Substrat eingesetzt.

Beispiele für Verbindungen der Formel IV sind Cumol, Cyclohexylbenzol, Cyclododecylbenzol, Ethylbenzol und 2-n-Butylbenzol.

Weitere, bevorzugte Substrate für das erfindungsgemäße Verfahren sind Verbindungen der Formeln V, VI, VII und VIII, wobei Ar einen aromatischen Kohlenwasserstoffrest bezeichnet.

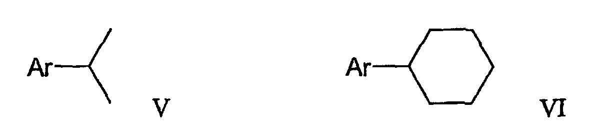

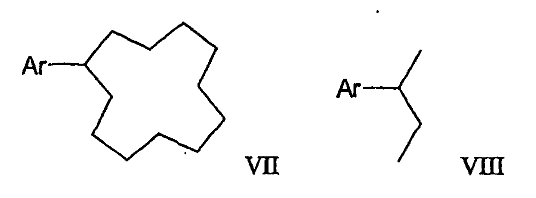

Die Oxidation dieser Kohlenwasserstoffe gemäß den Verfahren der Erfindung führt zu den entsprechenden Hydroperoxiden, Alkoholen, Aldehyden, Ketonen oder Carbonsäuren.

Der aromatische Kohlenwasserstoffrest Ar steht in den Formeln IV bis VIII für einen Phenylrest, Naphthylrest oder Diphenylrest.

Weiterhin kann als zu oxidierender Substrat ein cyclischer, gesättigter, substituierter oder unsubstituierter Kohlenwasserstoff der Formel X 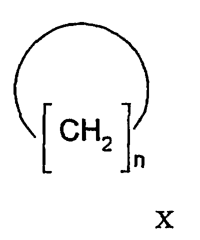 mit n = 3 bis 21 eingesetzt werden.

Außerdem kann als zu oxidierendes Substrat ein cyclischer, gesättigter, substituierter oder unsubstituierter Kohlenwasserstoff der Formel XI 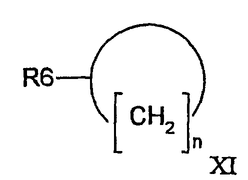 mit R⁶ = OH oder eine Alkylkette mit 1-10 C-Atomen und n = 3-21 eingesetzt werden.

Die erfindungsgemäße Reaktion kann in der Gasphase, in der Flüssigphase oder in der festen Phase ablaufen. Die Gasphase als Reaktionsmedium ist technisch dann relevant, wenn leicht flüchtige Stoffe wie Propan, Propen, Butan, Buten, Isobutan und Isobuten z.B. zu den entsprechenden Aldehyden oder Carbonsäuren oxidiert werden sollen. Die feste Phase kommt bei der Oberflächenmodifikation von festen Produkten wie Russen in Betracht. Man verwendet in diesem Fall beispielsweise beheizte Wirbelbettbäder oder Drehrohrkammeröfen. Besonders bevorzugt ist aber die Oxidation in flüssiger Phase. Das zu oxidierende Substrat kann auch selbst als Lösungsmittel dienen.

Durch das erfindungsgemäße Verfahren werden vermutlich zunächst die entsprechenden Hydroperoxide gebildet. Diese können - je nach Stabilität - isoliert und gezielt weiterverarbeitet werden, oder es erfolgt unter den gewählten Reaktionsbedingungen in situ eine weitere Umsetzung (d. h. Spaltung oder Umlagerung des Hydroperoxids) zu den Folgeprodukten (Aldehyde, Ketone oder Mono- oder Di- Carbonsäuren).

Das Reaktionsgemisch enthält einen Radikalstarter, der unter Bildung von Radikalen, d. h. den radikal-startenden Molekülen zerfällt, wie eine Peroxyverbindung oder eine Azoverbindung. Beispiele für solche Verbindungen sind Cumolhydroperoxid, Cyclohexylbenzolhydroperoxid, Cyclododecylbenzolhydroperoxid, 1,4-Di(2-neodecanoyl-peroxyisopropyl)benzol, Acetylcyclohexansulfonylperoxid, Cumylperoxyneodecanoat, Dicyclohexylperoxydicarbonat, Di(4-tert.-butylcyclohexyl)peroxydicarbonat, Di(2-ethylhexyl)peroxydicarbonat, Dimyristylperoxydicarbonat, Dicetylperoxydicarbonat, tert.-Butylperoxyneodecanoat, tert.-Amylperoxyneodecanoat, tert.-Amylperoxypivalat, tert.Butylperoxypivalat, Diisononanoylperoxid, Didecanoylperoxid, tert.-Butylperoxy-2-ethylhexanoat, tert.-Butylperoxyisononanoat, 2,2'-Di-ter.Butylperoxybutan, Di-tert.-Butylperoxybenzoat, Di-tert.-Butylperoxid, tert.-Butylhydroperoxid, 3,4-Dimethyl-3,4-diphenylhexan, Dibenzoylperoxid, 1,4-Di-tert.-butylperoxycyclohexan, tert.-Butylperoxyethylhexylcarbonat, 1,1-Di-tert.butylperoxycyclohexan, 2,2'-Azobis(2,4-dimethylvaleronitril, 2,2'-Azobis(2-methylpropionitril), 1,1'-Azobis(cyclohexancarbonitril) oder Cyclohexylhydroperoxid. Selbstverständlich können auch intermediär gebildete Peroxide und Azoverbindungen als Radikalstarter verwendet werden.

Bevorzugt wird ein Radikalstarter, der ein an ein sekundäres oder tertiäres Kohlenstoffatom gebundenes Sauerstoffatom enthält, besonders bevorzugt wird ein Radikalstarter, der sich vom Endprodukt ableitet und mindestens ein an ein primäres, sekundäres oder tertiäres Kohlenstoffatom gebundenes Sauerstoffatom enthält. Der Radikalstarter wird entweder separat zugegeben oder wie oben erwähnt während der Reaktion intermediär erzeugt oder er ist sogar, da eine Produktionsanlage nicht absolut gereinigt werden kann, in geringen Mengen aus vorausgegangenen Reaktionen noch vorhanden. Beispiele für solche Verbindungen sind Cumolhydroperoxid (1-Methyl-1-phenylethylhydroperoxid), Cyclohexylbenzolhydroperoxid (1-Phenylcyclohexylhydroperoxid), Cyclododecylbenzolhydroperoxid (1-Phenylcyclododecylhydroperoxid) und 2-n-Butylbenzolhydroperoxid (1-Methyl-1-phenylpropylhydroperoxid).

Die Konzentration des radikal-startenden Moleküls (z. B. Hydroxyradikal) ist im erfindungsgemässen Verfahren zu Beginn der Reaktion häufig geringer als die Konzentration des Katalysators. Es ist jedoch zu beachten, dass im Verlauf der Reaktion eine intermediäre Bildung dieser Verbindungen erfolgt, so dass sich die Konzentration der radikal-startenden Moleküle im Verlauf der Reaktion erhöht.

Das Mol-Verhältnis des Radikalstarters zum Katalysator kann 4 : 1, bevorzugt 3 : 1, ganz besonders bevorzugt 2 : 1 oder noch geringer wie 1 : 1 bis 0.5 : 1 betragen. Die Konzentration des radikal-startenden Moleküls ist von den Reaktionsparametern wie Temperatur oder Reaktionszeit abhängig.

Je geringer der Anteil des Radikalstarters ist, desto langsamer wird die Reaktion, da erst ein gewisser Anteil an radikal-startenden Molekülen gebildet werden muß. Die Menge an Radikalstarter kann auf Kosten der Selektivität noch weiter reduziert werden, wenn bei höherer Temperatur gearbeitet wird.

Das gebildete Oxidationsprodukt kann als solches isoliert werden, aber auch die direkte weitere Umsetzung dieser Verbindung zu einem weiteren Produkt ist möglich. Die Isolation des Produktes ist durch jedes gängige technische Verfahren wie z. B. Destillation oder Kristallisation möglich.

Das erfindungsgemäße Verfahren kann sowohl batchweise, im fedbatch als auch kontinuierlich durchgeführt werden.

Das erfindungsgemäße Verfahren kann unter Verwendung eines sauerstoffhaltigen Gases als Oxidationsmittel durchgeführt werden. Der Anteil des Sauerstoffs in dem Gas kann zwischen 5 bis 100 Vol% betragen. Bevorzugt wird Luftsauerstoff oder reiner Sauerstoff als Oxidationsmittel verwendet. Es ist in jedem Fall auf eine innige Vermischung der flüssigen und der gasförmigen Phase zu achten. Dies kann z. B. in Rührkesseln durch eine entsprechende Rührgeschwindigkeit oder durch Einbauten und in Rohrreaktoren mit Packungselementen sowie mit Blasensäulen erreicht werden.

Das erfindungsgemässe Verfahren kann sowohl mit geringem Unterdruck als auch unter Atmosphärendruck als auch unter erhöhtem Druck bis zu 100 bar durchgeführt werden. Bevorzugt wird ein Druck von 1 bar bis 50 bar, besonders bevorzugt wird ein Druck von 1 bar bis 20 bar.
Die folgenden Beispiele sollen die Erfindung näher erläutern, ohne ihren Schutzumfang einzuschränken.

Der Umsatz der Reaktion wurde zum einen durch die Titration des Hydroperoxids mit Iod und zum anderen durch die GC-Analyse mit einem internen Standard (Naphthalin) bestimmt. Die Selektivität der Reaktion wurde ebenfalls durch GC-Analyse mit einem internen Standard (ebenfalls Naphthalin) bestimmt.

### Beispiel 1 (erfindungsgemäss):

30 mmol Cyclohexylbenzol werden in einem Rundkolben mit aufgesetztem Rückflusskühler bei einer Temperatur von 110 °C mit 0,3 mmol N-Hydroxyphthalimid und 0,6 mmol 1-Cyclohexybenzylhydroperoxid versetzt. Das Reaktionsgemisch wird 8 Stunden bei der genannten Temperatur unter einer Sauerstoffatmosphäre von 1 bar gerührt. Es wird 1-Cyclohexylbenzolhydroperoxid mit einer Selektivität von 96,2 % bei einem Cyclohexylbenzol-Umsatz von 28,6 % erhalten.

### Beispiel 2 (nicht erfindungsgemäß, mit Co-Katalysator):

30 mmol Cyclohexylbenzol werden in einem Rundkolben mit aufgesetztem Rückflusskühler bei einer Temperatur von 110 °C mit 0,3 mmol N-Hydroxyphthalimid und 0,3 mmol Co(II)-Acetat versetzt. Das Reaktionsgemisch wird 8 Stunden bei der genannten Temperatur unter einer Sauerstoffatmosphäre von 1 bar gerührt. Es wird 1-Cyclohexylhenzolhydroperoxid mit einer Selektivität von 54,1 % bei einem Cyclohexylbenzol-Umsatz von 18,7 % erhalten.

### Beispiel 3 (erfindungsgemäss):

30 mmol Cumol werden in einem Rundkolben mit aufgesetztem Rückflusskühler bei einer Temperatur von 125 °C mit 0,3 mmol N-Hydroxyphthalimid und 0,6 mmol Cumolhydroperoxid versetzt. Das Reaktionsgemisch wird 8 Stunden bei der genannten Temperatur unter einer Sauerstoffatmosphäre von 1 bar gerührt. Es wird Cumolhydroperoxid mit einer Selektivität von 99,9 % bei einem Cumol-Umsatz von 30,8 % erhalten.

### Beispiel 4 (nicht erfindungsgemäß, mit Co-Katalysator):

30 mmol Cumol werden in einem Rundkolben mit aufgesetztem Rückflusskühler bei einer Temperatur von 125 °C mit 0,3 mmol N-Hydroxyphthalimid und 0,3 mmol Co(II)-Acetat versetzt. Das Reaktionsgemisch wird 8 Stunden bei der genannten Temperatur unter einer Sauerstoffatmosphäre von 1 bar gerührt. Es wird nicht das Zielprodukt sondern Acetophenon mit einer Selektivität von 58,7 %, 2-Phenyl-2-propanol (13,1 %) und Phenol (10,4%) bei einem Cumol-Umsatz von 49,3 % erhalten.

### Beispiel 5 (erfindungsgemäss):

30 mmol Cyclododecylbenzol werden in einem Rundkolben mit aufgesetztem Rückflusskühler bei einer Temperatur von 125 °C mit 0,3 mmol N-Hydroxyphthalimid und 0,6 mmol Cyclododecylbenzolhydroperoxid versetzt. Das Reaktionsgemisch wird 8 Stunden bei der genannten Temperatur unter einer Sauerstoffatmosphäre von 1 bar gerührt. Es wird Cyclododecylbenzolhydroperoxid mit einer Selektivität von 95,1 % bei einem Cyclododecylbenzol-Umsatz von 23,1 % erhalten.

### Beispiel 6 (nicht erfindungsgemäß, mit Co-Katalysator):

30 mmol Cyclododecylbenzol werden in einem Rundkolben mit aufgesetztem Rückflusskühler bei einer Temperatur von 125 °C mit 0,03 mmol N-Hydroxyphthalimid und 0,03 mmol Co(II)-Acetat versetzt. Das Reaktionsgemisch wird 8 Stunden bei der genannten Temperatur unter einer Sauerstoffatmosphäre von 1 bar gerührt. Es wird Cyclododecylbenzolhydroperoxid mit einer Selektivität von 59,1 % bei einem Cyclododecylbenzol-Umsatz von 7,3 % erhalten.

### Beispiel 7 (erfindungsgemäß):

30 mmol Ethylbenzol werden in einem Rundkolben mit aufgesetztem Rückflusskühler bei einer Temperatur von 125 °C mit 0,3 mmol N-Hydroxyphthalimid und 0,6 mmol Ethylbenzolhydraperoxid versetzt. Das Reaktionsgemisch wird 8 Stunden bei der genannten Temperatur unter einer Sauerstoffatmosphäre von 1 bar gerührt. Es wird Ethylbenzolhydroperoxid mit einer Selektivität von 99,9 % bei einem Ethylbenzol-Umsatz von 8 % erhalten.

### Beispiel 8 (nicht erfindungsgemäß, mit Co-Katalysator):

30 mmol Ethylbenzol werden in einem Rundkolben mit aufgesetztem Rückflusskühler bei einer Temperatur von 125 °C mit 0,3 mmol N-Hydroxyphthalimid und 0,3 mmol Co(II)-Acetat versetzt. Das Reaktionsgemisch wird 8 Stunden bei der genannten Temperatur unter einer Sauerstoffatmosphäre von 1 bar gerührt. Es wird Ethylbenzolhydroperoxid mit einer Selektivität von 22,4 % bei einem Ethylbenzol-Umsatz von 0,5 % erhalten.

### Beispiel 9 (erfindungsgemäß):

30 mmol Toluol werden in einem Rundkolben mit aufgesetztem Rückflusskühler bei einer Temperatur von 125 °C mit 0,3 mmol N-Hydroxyphthalimid und 0,6 mmol Cumolhydroperoxid versetzt. Das Reaktionsgemisch wird 8 Stunden bei der genannten Temperatur unter einer Sauerstoffatmosphäre von 1 bar gerührt. Es wird Benzylhydroperoxid mit einer Selektivität von 51,7 % bei einem Toluol-Umsatz von 8 % erhalten. Weitere Produkte sind Benzaldehyd (24,6 %) und Benzoesäure (23,6 %).

### Beispiel 10 (nicht erfindungsgemäß, mit Co-Katalysator):

30 mmol Toluol werden in einem Rundkolben mit aufgesetztem Rückflusskühler bei einer Temperatur von 125 °C mit 0,3 mmol N-Hydroxyphthalimid und 0,3 mmol Co(II)-Acetat versetzt. Das Reaktionsgemisch wird 8 Stunden bei der genannten Temperatur unter einer Sauerstoffatmosphäre von 1 bar gerührt. Es wird Benzylhydroperoxid mit einer Selektivität von 18,9 % bei einem Toluol-Umsatz von 3,7 % erhalten. Daneben gibt es eine Vielzahl weiterer Produkte, u.a. Benzaldehyd und Benzoesäure.

### Beispiel 11 (erfindungsgemäß):

In einem 2 1 -Glasbehälter mit innenliegender Glasfritte (zur Lufteinleitung) wurden 500 g eines Polyethylenwachses mit einem osmometrischen Molekulargewicht von 1450 und einer Säurezahl von 0 mg KOH/g Wachs bei 170 °C mit Luft (Durchsatz 6 l/min), unter Zusatz von 0,1 g N-Hydroxyphthalimid in der Schmelze oxidiert. Nach 3 Stunden war eine Säurezahl von 23 mg KOH/g Wachs erreicht. Das Produkt behielt seine weisse Eigenfarbe.

### Beispiel 12 (nicht erfindungsgemäß, mit Co-Katalysator):

In einem 2 1 -Glasbehälter mit innenliegender Glasfritte (zur Lufteinleitung) wurden 500 g eines Polyethylenwachses mit einem osmometrischen Molekulargewicht von 1450 und einer Säurezahl von 0 mg KOH/g Wachs bei 170 °C mit Luft (Durchsatz 6 l/min), unter Zusatz von 0,1 g N-Hydroxyphthalimid und 0,22g Co(II)-Acetat in der Schmelze oxidiert. Nach 6 Stunden war erst eine Säurezahl von 17 mg KOH/g Wachs erreicht. Das Produkt war gelbbraun verfärbt.

### Beispiel 13 (erfindungsgemäss):

200 mmol Cyclopentadecan werden in einem Rundkolben mit aufgesetztem Rückflusskühler bei einer Temperatur von 130 °C mit 2 mmol N-Hydroxyphthalimid und 4 mmol Cumolhydroperoxid versetzt. Durch das Reaktionsgemisch werden bei der genannten Temperatur 8 Stunden ca. 15 l/h Sauerstoff geleitet. Es wird Cyclopentadecanol mit einer Selektiviät von 20 % und Cyclopentadecanon mit einer Selektivität von 46 % bei einem Cyclopentadecan-Umsatz von 44 % erhalten.

### Beispiel 14 (erfindungsgemäss):

200 mmol Cyclopentadecan werden in einem Rundkolben mit aufgesetztem Rückflusskühler bei einer Temperatur von 130 °C mit 2 mmol N-Hydroxyphthalimid und 4 mmol Cumolhydroperoxid versetzt. Durch das Reaktionsgemisch werden bei der genannten Temperatur 8 Stunden ca. 15 l/h Luft geleitet. Es wird Cyclopentadecanol mit einer Selektiviät von 19 % und Cyclopentadecanon mit einer Selektivität von 48 % bei einem Cyclopentadecan-Umsatz von 46 % erhalten.

### Beispiel 16 (erfindungsgemäss):

200 mmol Cyclooctan werden in einem Rundkolben mit aufgesetztem Rückflusskühler bei einer Temperatur von 130 °C mit 2 mmol N-Hydroxyphthalimid und 4 mmol Cumolhydroperoxid versetzt. Durch das Reaktionsgemisch werden bei der genannten Temperatur 8 Stunden ca. 15 l/h Sauerstoff geleitet. Es wird Cyclooctanol mit einer Selektiviät von 17 % und Cyclooctanon mit einer Selektivität von 46 % bei einem Cyclooctan-Umsatz von 53 % erhalten.

### Beispiel 17 (erfindungsgemäss):

200 mmol Cyclooctan werden in einem Rundkolben mit aufgesetztem Rückflusskühler bei einer Temperatur von 130 °C mit 2 mmol N-Hydroxyphthalimid und 4 mmol Di-tert.-butylperoxid versetzt. Durch das Reaktionsgemisch werden bei der genannten Temperatur 8 Stunden ca. 15 l/h Luft geleitet. Es wird Cyclooctanol mit einer Selektiviät von 15 % und Cyclooctanon mit einer Selektivität von 43 % bei einem Cyclooctan-Umsatz von 51 % erhalten.

### Beispiel 18 (erfindungsgemäss):

200 mmol Cyclododecan werden in einem Rundkolben mit aufgesetztem Rückflusskühler bei einer Temperatur von 130 °C mit 2 mmol N-Hydroxyphthalimid und 4 mmol Cumolhydroperoxid versetzt. Durch das Reaktionsgemisch werden bei der genannten Temperatur 8 Stunden ca. 15 l/h Sauerstoff geleitet. Es wird Cyclododecanol mit einer Selektiviät von 17 % und Cyclododecanon mit einer Selektivität von 43 % bei einem Cyclododecan-Umsatz von 49 % erhalten.

### Beispiel 19 (erfindungsgemäss):

200 mmol Cyclododecan werden in einem Rundkolben mit aufgesetztem Rückflusskühler bei einer Temperatur von 130 °C mit 2 mmol N-Hydroxyphthalimid und 4 mmol Cumolhydroperoxid versetzt. Durch das Reaktionsgemisch werden bei der genannten Temperatur 8 Stunden ca. 15 l/h Luft geleitet. Es wird Cyclododecanol mit einer Selektiviät von 18 % und Cyclododecanon mit einer Selektivität von 42 % bei einem Cyclododecan-Umsatz von 47 % erhalten.

## Patentansprüche

1. Verfahren zur katalytischen Oxidation von gesättigten oder ungesättigten, substituierten oder unsubstituierten Kohlenwasserstoffen, Heterocyclen, Alkoholen, Ethern, Ketonen, Aldehyden, Aminen oder Russ (Substrat) zum entsprechenden Hydroperoxid, Alkohol, Keton, Carbonsäure oder Dicarbonsäure,
**dadurch gekennzeichnet,**
**daß** als Katalysator eine Verbindung der Formel I 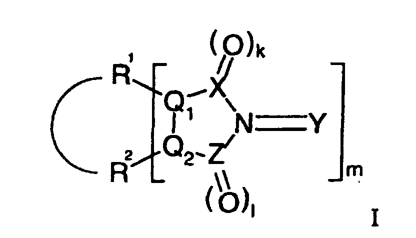 mit
R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können
Q₁, Q₂ = C, CH, N, CR⁵, jeweils gleich oder verschieden
X, Z = C, S, CH₂, jeweils gleich oder verschieden
Y = O, OH
k = 0, 1, 2
l = 0, 1, 2
m = 1 bis 3;
R⁵ : eine der Bedeutungen von R¹
unter Anwesenheit eines Radikalstarters eingesetzt wird, wobei das molare Verhältnis des Katalysators zum Kohlenwasserstoff zwischen 10⁻⁶ Mol-% und 2,5 Mol-% liegt.

2. Verfahren nach Anspruch 1,
**dadurch gekennzeichnet,**
**daß** als Katalysator eine Verbindung der Formel III 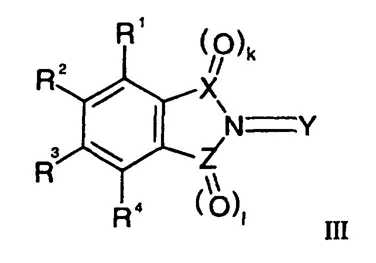 mit
R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹, R², R³ und R⁴ identische oder unterschiedliche Reste bezeichnen können, mit
X, Z = C, S, CH₂, jeweils gleich oder verschieden
Y = O, OH
k = 0, 1, 2
l = 0, 1, 2
eingesetzt wird.

3. Verfahren nach Anspruch 1,
**dadurch gekennzeichnet,**
**dass** als Katalysator ein Hydantoinderivat der Formel IX 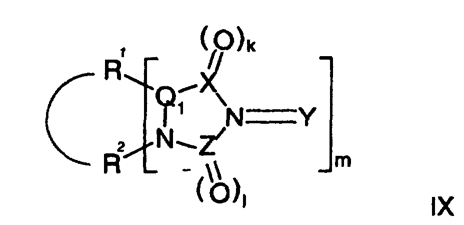 eingesetzt wird,
wobei Q¹, R¹, R², X, Z, k, l, m die im Anspruch 1 genannten Bedeutungen haben.

4. Verfahren nach einem der Ansprüche 1 bis 3,
**dadurch gekennzeichnet,**
**daß** als Radikalstarter eine Peroxyverbindung oder Azoverbindung verwendet wird.

5. Verfahren nach Anspruch 4,
**dadurch gekennzeichnet,**
**daß** Radikalstarter und Katalysator im Molverhältnis 4 : 1 eingesetzt werden.

6. Verfahren nach einem der Ansprüche 1 bis 5,
**dadurch gekennzeichnet,**
**daß** die katalytische Oxidation in der Flüssigphase bei einer Temperatur von 0 bis 500 °C durchgeführt wird.

7. Verfahren nach einem der Ansprüche 1 bis 6,
**dadurch gekennzeichnet,**
**daß** als Oxidationsmittel ein Gas mit 5 bis 100 Vol.-% Sauerstoff verwendet wird.

8. Verfahren nach einem der Ansprüche 1 bis 7,
**dadurch gekennzeichnet,**
**daß** die katalytische Oxidation unter einem Druck von 1 bis 100 bar durchgeführt wird.

9. Verfahren nach einem der Ansprüche 1 bis 8,
**dadurch gekennzeichnet,**
**daß** der zu oxidierende Kohlenwasserstoff Sauerstoff-, Stickstoff- und/oder Schwefelatome und/oder Cyanogruppen enthält.

10. Verfahren nach einem der Ansprüche 1 bis 9,
**dadurch gekennzeichnet,**
**daß** als zu oxidierendes Substrat ein Kohlenwasserstoff der Formel IV 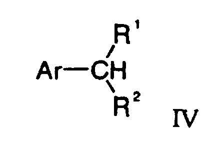 mit
R¹, R² = H, aliphatischer oder aromatischer Kohlenwasserstoffrest mit 1 bis 20 Kohlenstoffatomen, wobei R¹ und R² identische oder unterschiedliche Reste bezeichnen und R¹ und R² über eine kovalente Bindung miteinander verknüpft sein können und
Ar= aromatischer Kohlenwasserstoffrest
eingesetzt wird.

11. Verfahren nach einem der Ansprüche 1 bis 10,
**dadurch gekennzeichnet,**
**daß** als zu oxidierendes Substrat ein Kohlenwasserstoff der Formeln V, VI, VII und VIII 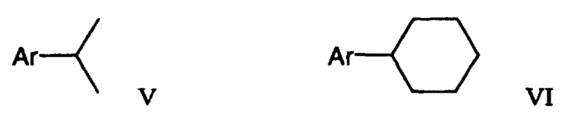 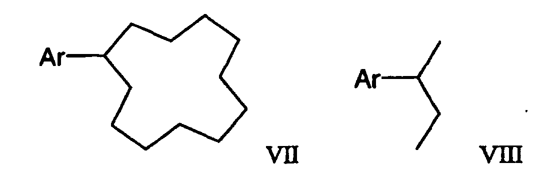 mit Ar= aromatischer Kohlenwasserstoffrest
eingesetzt werden.

12. Verfahren nach einem der Ansprüche 1 bis 9,
**dadurch gekennzeichnet,**
**dass** als zu oxidierendes Substrat ein cyclischer, gesättigter, substituierter oder unsubstituierter Kohlenwasserstoff der Formel X 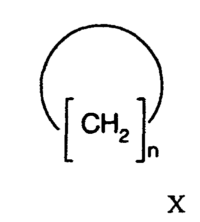 mit n = 3 bis 21 eingesetzt wird.

13. Verfahren nach einem der Ansprüche 1 bis 12,
**dadurch gekennzeichnet,**
**dass** als zu oxidierendes Substrat Aminocyclododecan, Butadien, Buten, Butylbenzol, Cumol, Cyclodecadien, Cyclodecan, Cyclodecen, Cyclododecadien, Cyclododecan, Cyclododecanol, Cyclododecanon, Cyclododecatrien, Cyclododecen, Cyclododecylbenzol, Cyclohexan, Cyclohexanol, Cyclohexen, Cyclohexanon, Cyclohepten, Cyclohexylbenzol, Cyclononen, Cyclooctadien, Cyclooctan, Cyclooctanol, Cyclooctanon, Cycloocten, Cyclopentadecan, Cyclopentadecanol, Cyclopentadecanon, Cyclopentadecatrien, Cyclopentadecen, Cycolopentadecadien, Decalin, Dicyclododecylether, Ethylbenzol, Isobutan, Isobuten, Isophoron, Isophoronderivate, meta-Xylol, ortho-Xylol, para-Xylol, Picolin, Propan, Propen, Tetralin, Toluol, Trimethylcyclohexan, Trimethylcyclohexanol, Trimethylcyclohexanon, Trivinylcyclohexan, jeweils als Reinstoff, in Lösung oder als Mischung eingesetzt wird.

14. Verfahren nach einem der Ansprüche 1 bis 9,
**dadurch gekennzeichnet,**
**dass** als zu oxidierendes Substrat ein cyclischer, gesättigter, substituierter oder unsubstituierter Kohlenwasserstoff der Formel XI 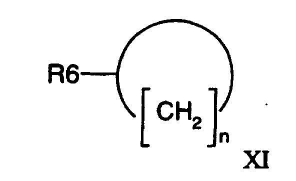 mit R⁶ = OH oder eine Alkylkette mit 1-10 C-Atomen .
und n = 3-21
eingesetzt wird.

15. Verfahren nach Anspruch 1,
**dadurch gekennzeichnet,**
**dass** als zu oxidierendes Substrat ein Wachs natürlicher oder synthetischer Herkunft eingesetzt wird.

16. Verfahren nach einem der Ansprüche 1 bis 15,
**dadurch gekennzeichnet,**
**dass** die katalytische Oxidation in Gegenwart von Ammoniak durchgeführt wird.

## Claims

1. A process for catalytically oxidizing saturated or unsaturated substituted or unsubstituted hydrocarbons, heterocycles, alcohols, ethers, ketones, aldehydes, amines or soot (substrate) to the corresponding hydroperoxide, alcohol, ketone, carboxylic acid or dicarboxylic acid, **characterized in that**
the catalyst used is a compound of the formula I 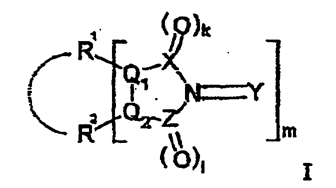 where
R¹, R² = H, aliphatic or aromatic alkoxy radical, carboxyl radical, alkoxycarbonyl radical or hydrocarbon radical, each of which has 1 to 20 carbon atoms, SO₃H, NH₂, OH, F, Cl, Br, I and/or NO₂ where R¹ and R² are identical or different radicals, or R¹ and R² can be linked to one another via a covalent bond
Q₁, Q₂ = C, CH, N, CR⁵, identical or different in each case
X, Z = C, S, CH₂, identical or different in each case
Y = O, OH
k = 0, 1, 2
l = 0, 1, 2
m = 1 to 3;
R⁵ : one of the meanings of R¹
is used in the presence of a free-radical initiator, the molar ratio of the catalyst to the hydrocarbon being from 10⁻⁶ mol% to 2.5 mol%.

2. A process according to claim 1, **characterized in that**
the catalyst used is a compound of the formula III 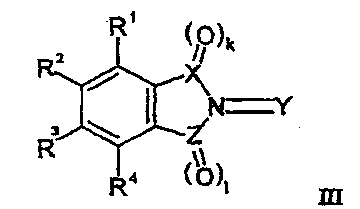 where
R¹, R² = H, aliphatic or aromatic alkoxy radical, carboxyl radical, alkoxycarbonyl radical or hydrocarbon radical, each of which has 1 to 20 carbon atoms, SO₃H, NH₂, OH, F, Cl, Br, I and/or NO₂ where R¹, R², R³ and R⁴ can be identical or different radicals, where
X, Z = C, S, CH₂ are identical or different in each case
Y = O, OH
k = 0, 1, 2
l = 0, 1, 2.

3. A process according to claim 1, **characterized in that**
the catalyst used is a hydantoin derivative of the formula IX 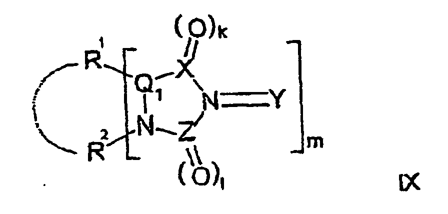 where Q¹, R¹, R², X, Z, k, l, m have the meanings specified in claim 1.

4. A process according to any one of claims 1 to 3,
**characterized in that** the free-radical initiator is a peroxy compound or azo compound.

5. A process according to claim 4,
**characterized in that** free-radical initiator and catalyst are used in a molar ratio of 4:1.

6. A process according to any one of claims 1 to 5,
**characterized in that** the catalytic oxidation is carried out in the liquid phase at a temperature of 0 to 500°C.

7. A process according to any one of claims 1 to 6,
**characterized in that** the oxidizing agent is a gas containing 5 to 100% by volume of oxygen.

8. A process according to any one of claims 1 to 7,
**characterized in that** the catalytic oxidation is carried out under a pressure of 1 to 100 bar.

9. A process according to any one of claims 1 to 8,
**characterized in that** the hydrocarbon to be oxidized contains oxygen, nitrogen and/or sulphur atoms and/or cyano groups.

10. A process according to any one of claims 1 to 9,
**characterized in that** the substrate to be oxidized is a hydrocarbon of the formula IV 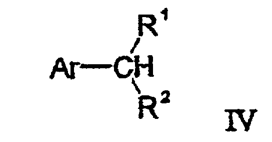 where
R¹, R² = H, aliphatic or aromatic hydrocarbon radical having 1 to 20 carbon atoms, where R¹ and R² are identical or different radicals and R¹ and R² can be linked to one another via a covalent bond and
Ar = aromatic hydrocarbon radical.

11. A process according to any one of claims 1 to 10,
**characterized in that** the substrate to be oxidized is a hydrocarbon of the formula- V, VI, VII or VIII 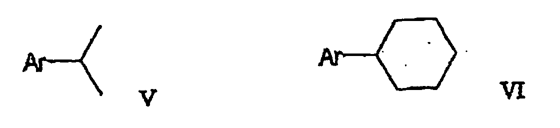 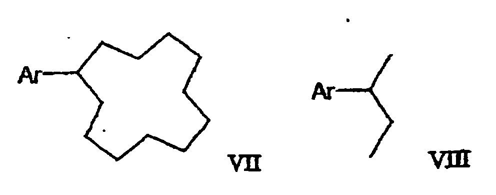 where Ar = aromatic hydrocarbon radical.

12. A process according to any one of claims 1 to 9,
**characterized in that** the substrate to be oxidized is a cyclic, saturated, substituted or unsubstituted hydrocarbon of the formula X 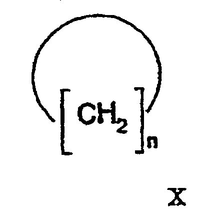 where n = 3 to 21.

13. A process according to any one of claims 1 to 12,
**characterized in that** the substrate to be oxidized is aminocyclododecane, butadiene, butene, butylbenzene, cumene, cyclodecadiene, cyclodecane, cyclodecene, cyclododecadiene, cyclododecane, cyclododecanol, cyclododecanone, cyclododecatriene, cyclododecene, cyclododecylbenzene, cyclohexane, cyclohexanol, cyclohexene, cyclohexanone, cycloheptene, cyclohexylbenzene, cyclononene, cyclooctadiene, cyclooctane, cyclooctanol, cyclooctanone, cyclooctene, cyclopentadecane, cyclopentadecanol, cyclopentadecanone, cyclopentadecatriene, cyclopentadecene, cyclopentadecadiene, decalin, dicyclododecyl ether, ethylbenzene, isobutane, isobutene, isophorone, isophorone derivatives, meta-xylene, ortho-xylene, para-xylene, picoline, propane, propene, tetralin, toluene, trimethylcyclohexane, trimethylcyclohexanol, trimethylcyclohexanone, trivinylcyclohexane, in each case as pure substance, in solution or as a mixture.

14. A process according to any one of claims 1 to 9,
**characterized in that** the substrate to be oxidized is a cyclic, saturated, substituted or unsubstituted hydrocarbon of the formula XI 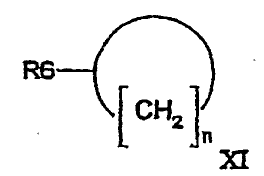 where R⁶ = OH or an alkyl chain having 1-10 carbon atoms
and n = 3-21.

15. A process according to claim 1,
**characterized in that** the substrate to be oxidized is a wax of natural or synthetic origin.

16. A process according to any one of claims 1 to 15,
**characterized in that** the catalytic oxidation is carried out in the presence of ammonia.

## Revendications

1. Procédé d'oxydation catalytique d'hydrocarbures, de corps hétérocycliques, d'alcools, d'éthers, de cétones, d'aldéhydes, d'amines ou de suie (substrat), saturés ou non saturés, substitués ou non substitués, en hydroperoxyde, alcool, cétone, acide carboxylique ou acide dicarboxylique correspondants,
**caractérisé en ce qu'**
on utilise comme catalyseur un composé de formule I 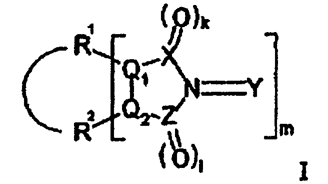 avec R¹, R² = H, radical alcoxy aliphatique ou aromatique, radical carboxyle, radical alcoxycarbonyle ou radical hydrocarbure, chacun avec 1 à 20 atomes de carbone, SO₃H, NH₂, OH, F, Cl, Br, I et/ou NO₂, avec R¹ et R² désignant des radicaux identiques ou différents, ou bien R¹ et R² pouvant être liés ensemble par une liaison covalente,
Q₁, Q₂ = C, CH, N, CR⁵, chaque fois identiques ou différents
X, Z = C, S, CH₂, chaque fois identiques ou différents
Y = O, OH
k = 0, 1, 2
l = 0, 1, 2
m = 1 à 3
R⁵ : l'une des significations de R¹
en présence d'un amorceur de radicaux, le rapport molaire du catalyseur à l'hydrocarbure se situant entre 10⁻⁶ et 2,5 % molaire.

2. Procédé selon la revendication 1
**caractérisé en ce qu'**
on utilise comme catalyseur un composé de formule III 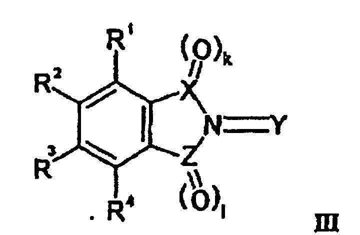 avec R¹, R² = H, radical alcoxy aliphatique ou aromatique, radical carboxyle, radical alcoxycarbonyle ou radical hydrocarbure, chacun avec 1 à 20 atomes de carbone, SO₃H, NH₂, OH, F, Cl, Br, I et/ou NO₂, avec R¹, R², R³ et R⁴ pouvant désigner des radicaux identiques ou différents, et
X, Z = C, S, CH₂, chaque fois identiques ou différents
Y = O, OH
k = 0, 1, 2
l = 0, 1, 2

3. Procédé selon la revendication 1
**caractérisé en ce qu'**
on utilise comme catalyseur un dérivé d'hydantoïne de formule IX 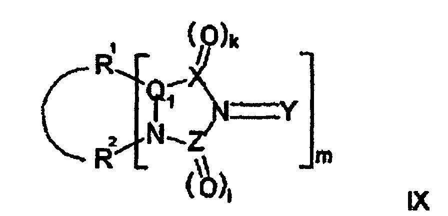 Q¹, R¹, R², X, Z, k, l et m ayant les significations données dans la revendication 1.

4. Procédé selon l'une quelconque des revendications 1 à 3,
**caractérisé en ce qu'**
on utilise comme amorceur de radicaux un composé de peroxyde ou un composé azo.

5. Procédé selon la revendication 4,
**caractérisé en ce que**
l'amorceur de radicaux et le catalyseur sont utilisés dans le rapport molaire 4/ 1.

6. Procédé selon l'une quelconque des revendications 1 à 5,
**caractérisé en ce qu'**
on effectue l'oxydation catalytique dans la phase liquide à une température de 0 à 500°C.

7. Procédé selon l'une quelconque des revendications 1 à 6,
**caractérisé en ce qu'**
on utilise comme agent d'oxydation un gaz avec de 5 à 100 % en volume d'oxygène.

8. Procédé selon l'une quelconque des revendications 1 à 7,
**caractérisé en ce qu'**
on effectue l'oxydation catalytique sous une pression de 1 à 100 bars.

9. Procédé selon l'une quelconque des revendications 1 à 8,
**caractérisé en ce que**
l'hydrocarbure à oxyder contient des atomes d'oxygène, d'azote et/ou de soufre et/ou des groupes cyano.

10. Procédé selon l'une quelconque des revendications 1 à 9,
**caractérisé en ce qu'**
on utilise comme substrat à oxyder un hydrocarbure de formule IV 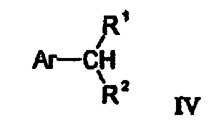 avec R¹, R² = H, radical hydrocarbure aliphatique ou aromatique avec de 1 à 20 atomes de carbone, R¹ et R² désignant des radicaux identiques ou différents et R¹ et R² pouvant être liés ensemble par une liaison covalente, et
Ar = radical hydrocarbure aromatique utilisé comme substrat.

11. Procédé selon l'une quelconque des revendications 1 à 10,
**caractérisé en ce qu'**
on utilise comme substrat à oxyder un hydrocarbure de formules V, VI, VII et VIII

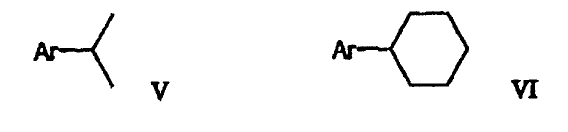

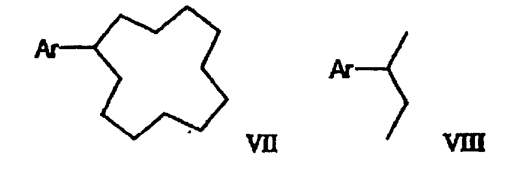

12. Procédé selon l'une quelconque des revendications 1 à 9
**caractérisé en ce qu'**
on utilise comme substrat à oxyder un hydrocarbure cyclique, saturé, substitué ou non substitué, de formule X 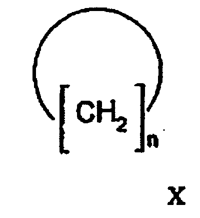 avec n = de 3 à 21.

13. Procédé selon l'une quelconque des revendications 1 à 12,
**caractérisé en ce qu'**
on utilise comme substrat à oxyder les substances suivantes : aminocyclododécane, butadiène, butène, butylbenzène, cumène, cyclodécadiène, cyclodécane, cyclodécène, cyclododécadiène, cyclododécane, cyclododécanol, cyclohexane, cyclohexanol, cyclohexène, cyclohexanone, cyclododécanone, cyclododécatriène, cyclododécène, cyclododécylbenzène, cycloheptène, cyclohexylbenzène, cyclononène, cyclo-octadiène, cyclooctane, cyclo-octanol, cyclo-octanone, cyclo-octène, cyclopentadécane, cyclopentadécanol, cyclopentadécanone, cyclopentadécatriène, cyclopentadécène, cyclopentadécadiène, décaline, dicyclododécyléther, éthylbenzène, isobutane, isobutène, isophorone, dérivés d'isophorone, méta-xylol, ortho-xylol, para-xylol, picohne, propane, propène, tétraline, toluène, triméthylcyclohexane, triméthylcyclohexanol, triméthylcyclohexanone, trivinylcyclohexane, chacun comme matière pure, en solution ou comme mélange.

14. Procédé selon l'une quelconque des revendications 1 à 9,
**caractérisé en ce qu'**
on utilise comme substrat oxydant un hydrocarbure cyclique, saturé ou non substitué, de formule XI 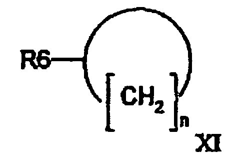 avec R⁶ = OH ou une chaîne alkyle avec de 1 à 10 atomes de carbone
et n = de 3 à 21.

15. Procédé selon la revendication 1,
**caractérisé en ce qu'**
on utilise comme substrat à oxyder une cire de provenance naturelle ou synthétique.

16. Procédé selon l'une quelconque des revendications 1 à 15,
**caractérisé en ce que**
l'oxydation catalytique s'effectue en présence d'ammoniac.
